(19) Europäisches Patentamt
European Patent Office
Office européen des brevets

(11) Publication number: 0 063 238 B1

(12) EUROPEAN PATENT SPECIFICATION

(45) Date of publication of patent specification: 29.08.84

(51) Int. Cl.$^3$: A 23 K 1/17, A 61 K 31/35, C 07 D 309/10

(21) Application number: 82102161.5

(22) Date of filing: 17.03.82

# (54) An efficiency-improving agent for feed and a preventive and treating agent for coccidiosis.

(30) Priority: 21.04.81 JP 59154/81

(43) Date of publication of application: 27.10.82 Bulletin 82/43

(45) Publication of the grant of the patent: 29.08.84 Bulletin 84/35

(84) Designated Contracting States: AT BE CH DE FR GB IT LI LU NL SE

(56) References cited:
DE-A-2 802 455
US-A-4 192 887
US-A-4 303 647

(73) Proprietor: KAKEN CHEMICAL COMPANY, LTD.
No. 28-8, 2-chome, Honkomagome Bunkyo-Ku
Tokyo 113 (JP)

(72) Inventor: Kusakabe, Yoko KAKEN CHEMICAL COMPANY, LIMITED
28-8 2-chome, Honkomagome Bunkyo-ku
Tokyo (JP)
Inventor: Mizuno, Taku KAKEN CHEMICAL COMPANY, LIMITED
28-8 2-chome, Honkomagome Bunkyo-ku
Tokyo (JP)
Inventor: Seino, Akio KAKEN CHEMICAL COMPANY, LIMITED
28-8 2-chome Honkomagome Bunkyo-ku
Tokyo (JP)

(74) Representative: Vossius Vossius Tauchner Heunemann Rauh
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 063 238 B1

Courier Press, Leamington Spa, England.

**Description**

FIELD OF THE INVENTION

The present invention relates to an efficiency-improving agent for a livestock or poultry feed or a preventive and treating agent for livestock or poultry coccidiosis containing antibiotics.

BACKGROUND OF THE INVENTION

Attempts have already been made widely to promote the growth of and improve the fecundity of poultry and livestock by the addition of an antibiotic to feed. In the case of an antibiotic used in both man and animals, however, man is subject to deleterious effects such as the emergence of resistant bacteria and so forth since he is bound to simultaneously ingest the antibiotic that has remained and accumulated in the animal body when he eats the animal.

SUMMARY OF THE INVENTION

Therefore, it is an object of the present invention to provide an efficiency-improving agent for a livestock or poultry feed or a preventive and treating agent for livestock or poultry coccidiosis which is free of the above-described drawbacks.

As a result of extensive investigations it is found that antibiotics 5057A and 5057B can remarkably improve the efficiency of the utilization of a livestock and poultry feed and have a preventive and treating activity against livestock and poultry coccidiosis.

The present invention is based on this finding and provides an efficiency-improving agent for a livestock or poultry feed or a preventive and treating agent for livestock or poultry coccidiosis which contains antibiotic 5057A or its physiologically acceptable salt and/or antibiotic 5057B or its physiologically acceptable salt as an effective ingredient.

DETAILED DESCRIPTION OF THE INVENTION

Antibiotics 5057A and 5057B are the ones found in a culture broth of a Streptomyces sp. 5057 strain (FERM BP 62); method for the preparation of the antibiotics 5057A and 5057B and physical and chemical characteristics thereof are described in Japanese Patent Publication No. 8641/1979 and EP-A-0064555, respectively. Both of the antibiotics are produced by cultivating a Streptomyces s.p. 5057 strain according to the usual method. The A and B substances can be isolated according to the ordinary method; they can also be obtained as a mixture.

The antibiotics 5057A and 5057B both belong to an antibiotic of the polyether series and are known to have an excellent antibacterial activity.

The effective ingredient of the composition of the present invention, antibiotic 5057A and/or 5057B can be used in the form of a purified preparation or a crude product, or as cells containing the effective ingredient. The antibiotics 5057A and 5057B may be in the form of a physiologically acceptable salt such as sodium and potassium salts.

The efficiency-improving agent for feed or preventive and treating agent for coccidiosis is used as a preparation, such as powders, tablets, capsules, granules and pills, that is prepared by either mixing or not mixing.

Antibiotic 5057A and/or 5057B with a physiologically acceptable solid or liquid diluent; however, usually it is used by directly incorporating it into feed or drinking water or by adding its dispersion in a diluent to feed or drinking water. It is, when added to feed, used preferably as, for example, a premix prepared from it. The premix is obtained by mixing a purified product or a crude product of the effective ingredient, or cells containing it with a physiologically acceptable solid or liquid carrier. As a solid carrier, there are used, for example, flour, soybean powders, rice bran, corn powders, starch, glucose, yeast, fish meal, talc, and diatomaceus earth, etc. As a liquid carrier, there are used, for example, physiological saline solution, distilled water, and a physiologically acceptable organic solvent, etc.

Further, suitable supplementary agents or additives such as an emulsifying agent, dispersant, suspending agent, moistening agent, concentrating agent, gelation agent, antimicrobial agent, antiseptic agent, enzyme preparation, antibiotic, and lactic acid preparation, etc. can also be used in admixture with the foregoing preparations.

The concentration of the effective ingredient in the premix can suitably be adjusted depending on the kind of livestock or poultry.

The concentration for use of the efficiency-improving agent for feed of the present invention varies according to the kind and age of livestock or poultry; for example, in the case of poultry such as a domestic fowl, quail, turkey, guinea fowl, duck and, goose, it is administered as a feed containing usually 5—200 ppm, preferably 10—100 ppm, of the effective ingredient. There are used a feed containing usually 5—200 ppm, preferably 10—100 ppm, of the effective ingredient in the case of swine and a feed containing usually 1—100 ppm, preferably 2—50 ppm, of the effective ingredient in the case of a ruminant such as cattle, sheep, and goat. As a preventive and treating agent for coccidiosis, there is used a feed containing 10—250 ppm, preferably 20—100 ppm, of the effective ingredient.

The effective ingredient of the present invention, antibiotics 5057A and 5057B, are excellent in

that it is effective in not only improving the efficiency of feed but also promoting the growth; it also has a special feature of low toxicity.

The present agent, when administered to poultry, can improve the efficiency of feed, prevent the occurrence of coccidiosis which is dreadful for poultry, and cure the disease. On its administration to swine, it not only improves the efficiency of the feed utilization but also prevents and cures diarrhea from which a young pig is liable to suffer. On its administration to a ruminant, an increased ratio of propionic acid to acetic and butyric acids in the rumen of the animal is observed. In general the utilization ratio of propionic acid is said to be large in the stomach of a ruminant, although a ruminant also utilizes acetic and butyric acid compounds in its stomach. It is considered that the production of propionic acid is promoted in the rumen by the administration of the present agent, and as a result the utilization efficiency of feed is improved.

The present invention will be explained in detail by the following examples and test examples.

Example 1

| | |
|---|---|
| 5057A | 1% |
| cornstarch | 99% |

The two substances are crushed and mixed uniformly to make a premix containing 1% of 5057A.

Example 2

| | |
|---|---|
| 5057B | 5% |
| soybean powders | 95% |

A premix containing 5% of 5057B is prepared in the same manner as in Example 1.

Example 3

| | |
|---|---|
| 5057A | 0.5% |
| 5057B | 0.5% |
| soybean cake | 99 % |

A premix containing 0.5% of 5057A and 0.5% of 5057B is prepared in the same manner as in Example 1.

Example 4

| | |
|---|---|
| crude product of 5057A (40% purity) | 2.5% |
| crude product of 5057B (40% purity) | 2.5% |
| bran | 95% |

The substances are crushed and mixed uniformly to make a premix containing 1.0% of 5057A and 1.0% of 5057B.

Example 5

Dried cultured cells (1000 g) containing 1.12% of 5057A and 0.86% of 5057B are crushed and used, as they are, as a premix.

Test Example 1
Feed efficiency test on domestic fowls
Domestic fowls tested: species used exclusively for broiler (Aber Acres species)

Basal feed:

| | |
|---|---|
| yellow corn | 42% |
| flour | 25% |
| soybean cake | 20% |
| fish cake | 8% |
| alfalfa meal | 3% |
| tricalcium phosphate | 1% |
| calcium carbonate | 0.25% |
| table salt | 0.45% |
| vitamin | 0.15% |
| mineral | 0.1% |
| methionine | 0.05% |

Method:

Fifty newborn chickens were divided into 5 groups of 10 each in such a way that the average body weight is the same in each group. Premixes of 5057A and 5057B were added to the foregoing basal feed so that the contents of 5057A and 5057B are 40 ppm and 80 ppm, respectively. This feed was administered to the animals over the period of 70 days, and the body weight and the ingested amount of the feed were measured.

The results are shown in Table 1 below.

TABLE 1

| Group | Substance added to the feed to be administered | Added amount (ppm) | The average body weight at the start (kg) | The average increase in the body weight during the test period (kg) | The average ingested amount of feed during the test period (kg) | The rate of feed requirement | The improved ratio of the rate of feed requirement (%) |
|---|---|---|---|---|---|---|---|
| 1 | 5057A | 40 | 43.5 | 2.54 | 5.59 | 2.201 | 4.3 |
| 2 | " | 80 | 43.1 | 2.55 | 5.63 | 2.218 | 4.0 |
| 3 | 5057B | 40 | 43.5 | 2.67 | 5.74 | 2.150 | 6.6 |
| 4 | " | 80 | 43.2 | 2.62 | 5.67 | 2.164 | 6.0 |
| 5 | no addition | 0 | 43.6 | 2.46 | 5.66 | 2.301 | — |

$$\text{The rate of feed requirement} = \frac{\text{The average ingested amount of feed during the test period}}{\text{The average increase in the body weight during the test period}}$$

$$\text{The improved ratio of the rate of feed requirement (\%)} = 1 - \left( \frac{\text{The rate of feed requirement in the case of the addition of the agent}}{\text{The rate of feed requirement in the case of no addition of the agent}} \right) \times 100$$

The administration of the feed to which the present substances had been added improved the rate of feed requirement by 4—6.5% and the increase in the body weight by 3—9%.

Test Example 2
Feed efficiency test on swine

Swine tested: landrace

Basal feed:

a. the start of the test — 4 weeks

| | |
|---|---|
| cereals (corn, wheat, barley) | 60% |
| soybean oil cake | 15% |
| feed of an animal nature (defatted powdered milk, fish meal) | 15% |
| others (enzyme, calcium carbonate, calcium phosphate, table salt, etc.) | 10% |

b. 4—12 weeks

| | |
|---|---|
| cereals (corn, milo, wheat) | 78% |
| soybean oil cake | 13% |
| fish meal | 5% |
| others (table salt, calcium carbonate, calcium phosphate, etc.) | 4% |

Method:

Thirty young pigs were divided into 5 groups of 6 each in such a way that the average body weight is the same in each group. To the basal feed were added 0,25, and 50 ppm each of 5057A and 5057B. The resulting basal feeds were administered over the period of 12 weeks and the body weight and the ingested amount of the feeds were measured. The total number of days that the pigs suffered from diarrhea during the period of 4 weeks was counted.

The results are shown in Table 2 below.

TABLE 2

| Group | Substance added to the feed to be administered | Added amount (ppm) | The average body weight at the start (kg) | The average increase in the body weight during the test period (kg) | The average ingested amount of feed during the test period (kg) | The rate of feed requirement | The improved ratio of the rate of feed requirement (%) | The total number of days of diarrheal stool in each group / The total number of days during the test period in each group |
|---|---|---|---|---|---|---|---|---|
| 1 | 5057A | 25 | 6.83 | 45.35 | 106.7 | 2.353 | 4.3 | 21/168 |
| 2 | " | 50 | 6.80 | 50.93 | 116.5 | 2.287 | 7.0 | 11/168 |
| 3 | 5057B | 25 | 6.82 | 47.62 | 111.8 | 2.348 | 4.6 | 18/168 |
| 4 | " | 50 | 6.78 | 51.26 | 116.0 | 2.263 | 8.0 | 6/168 |
| 5 | no addition | 0 | 6.83 | 43.70 | 107.5 | 2.460 | — | 57/168 |

The administration of the feeds to which the present substances had been added improved the rate of the feed utilization by 4—8% and the increased amount of the body weight by 4—17%. A marked decrease in the occurrence of swine diarrhea was also observed.

Test Example 3

Feed efficiency test on a ruminant

Cattle tested: Holstein

Basal feed:

Concentrated feed:

| | |
|---|---|
| New king beef, later period (prepared by Kumiai Feed Co., Ltd.) | 66% |
| flake corn | 17% |
| flake barley | 17% |

Method:

Twenty five castrated Holstein oxen 9 months of age were divided into 5 groups of 5 each in such as way that the average body weight is almost the same in each group. Concentrated basal feeds to which 0, 10 and 20 ppm each of 5057A and 5057B had been added were each administered over the period of 240 days. As a crude feed, 1 kg per animal of straw of rice plants were administered.

The results are shown in Table 3 below.

TABLE 3

| Group | Substance added to the feed to be administered | Added amount (ppm) | The average body weight at the start (kg) | The average increase in the body weight during the test period (kg) | The average ingested amount of feed during the test period (kg) | The rate of feed requirement | The improved ratio of the rate of concentrated feed requirement (%) |
|---|---|---|---|---|---|---|---|
| 1 | 5057A | 10 | 335 | 271 | 2413 | 8.904 | 10.2 |
| 2 | " | 20 | 325 | 280 | 2365 | 8.446 | 14.8 |
| 3 | 5057B | 10 | 345 | 273 | 2406 | 8.813 | 11.1 |
| 4 | " | 20 | 327 | 284 | 2354 | 8.289 | 16.4 |
| 5 | no addition | 0 | 337 | 262 | 2598 | 9.916 | — |

The administration of the feeds to which the present substances had been improved the utilization rate of the concentrated feed by 10—17% and the increased amount of the body weight by 3—8%. The gastric juice from the rumen of the oxen tested was collected and measured for the concentrations of acetic and propionic acids, thus the ratio between them being calculated. The results are shown in Table 4 below.

TABLE 4

| Substance added to the feed to be administered | Amount added (ppm) | The propionic acid concentration/The acetic acid concentration (the average value): Before the start of the administration | 1 day after the administration | 21 days after the administration | 56 days after the administration |
|---|---|---|---|---|---|
| 5057A | 10 | 0.51 | 0.80 | 0.73 | 0.78 |
| | 20 | 0.54 | 0.83 | 0.96 | 0.86 |
| 5057B | 10 | 0.54 | 0.79 | 0.76 | 0.77 |
| | 20 | 0.52 | 0.83 | 1.00 | 0.93 |
| — | 0 | 0.56 | 0.51 | 0.63 | 0.58 |

It can be learned that the administration of 5057A or 5057B increases the ratio between propionic acid/acetic acid in the gastric juice in the rumen as compared with the case of no addition. This fact can be considered to be responsible for the improvement in the efficiency rate of the feed utilization.

Test Example 4

The preventive and treating activity against coccidiosis in a domestic fowl.

Domestic fowls tested: white leghorns

Basal feed:

| | |
|---|---|
| corn | 47% |
| soybean powders | 15% |
| soybean cake | 15% |
| flour | 10% |
| fish meal | 7% |
| alfalfa meal | 3.8% |
| tricalcium phosphate | 1% |
| calcium carbonate | 0.3% |
| table salt | 0.5% |
| vitamin | 0.2% |
| mineral | 0.1% |
| methionine | 0.1% |

Method:

Fifty male white leghorn chickens 14 days of age were divided into 5 groups of 10 each. To the basal feed were added 50 and 100 ppm each of 5057A and 5057B. Infection with coccidiosis was

done by inoculating 7 x $10^{14}$ per chicken of spore-forming oocysts of *Eimeria tenellum* directly p.o. into the crop of the chickens.

Groups 1—4 were reared with the feeds containing predetermined concentrations of the 5057 substances, starting from 1 day before the inoculation with oocysts. Group 5 is an infection control that was inoculated with oocysts and reared with the basal feed containing no agents tested and Group 6 is a noninfection control. After 7 days after the inoculation with oocysts, the number of oocysts (OPG) in 1 g of stool was counted.

The results are shown in Table 5 below.

$$\text{Death rate} = \frac{\text{Dead chickens upon termination of the test}}{\text{Number of chickens on the day of the start of the test}} \times 100$$

TABLE 5

| Group | Substance given | Amount of the administration (ppm) | Death rate (%) | Relation rate of the increase in the body weight (%) | OPG (x $10^4$) |
|---|---|---|---|---|---|
| 1 | 5057A | 50 | 0 | 104 | 0.01 |
| 2 | " | 100 | 0 | 103 | 0 |
| 3 | 5057B | 50 | 0 | 106 | 0 |
| 4 | " | 100 | 0 | 106 | 0 |
| 5 | Infection control | 0 | 50 | 79 | 87 |
| 6 | Noninfection control | 0 | 0 | 100 | 0 |

From the test results, it can be learned that antibiotics 5057A and 5057B have a preventive and treating activity against coccidiosis.

Test Example 5

Acute toxicity

An acute toxicity test was carried out with mice. As a result, the $LD_{50}$ value of the 5057A and 5057B substances is 50 mg/kp i.p. and 50 mg/kg or more p.o.

Antibiotic

5057—A

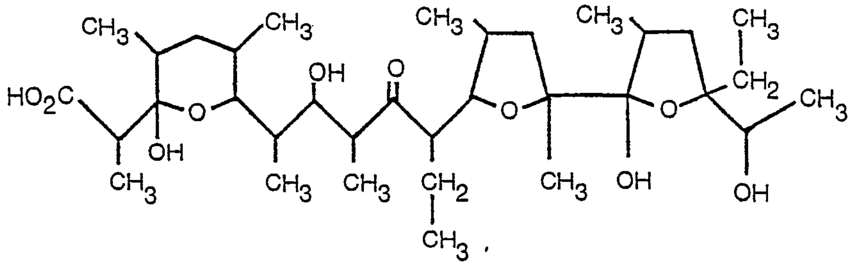

Antibiotic

5057—B

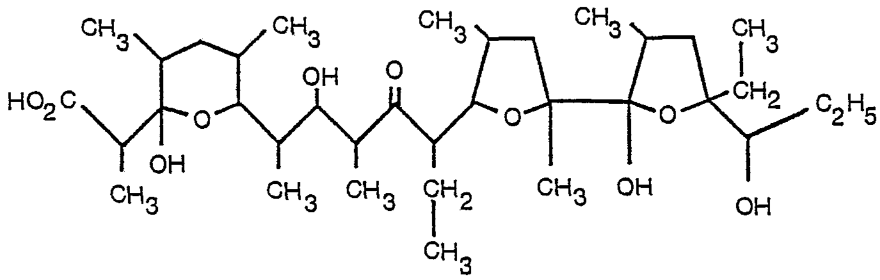

**Claims for the Contracting States: BE CH DE FR GB IT LI LU NL SE**

1. An efficiency-improving agent for a livestock or poultry feed or a preventive and treating agent for livestock or poultry coccidiosis, characterized in that it contains antibiotic 5057A or its physiologically acceptable salt and/or antibiotic 5057B or its physiologically acceptable salt as an effective ingredient.

2. The efficiency-improving agent for a livestock or poultry feed according to Claim 1 in which the amount of the effective ingredient is 5—200 ppm.

3. The preventive and treating agent for livestock or poultry coccidiosis according to Claim 1 in which the amount of the effective ingredient is 10—250 ppm.

4. The efficiency-improving agent for a livestock or poultry feed or a preventive and treating agent for livestock or poultry coccidiosis according to Claim 1, 2 or 3 in which the feed or agent is in the form of powders, tablets, capsules, granules or pills.

5. The efficiency-improving agent for a livestock or poultry feed or a preventive and treating agent for livestock or poultry coccidiosis according to Claim 1, 2 or 3 in which the agent is a premix comprising a purified product or a crude product of the effective ingredient, or cells containing it and a physiologically acceptable solid or liquid carrier.

6. The efficiency-improving agent for a livestock or poultry feed according to Claim 5 in which the solid carrier is flour, soybean powders, rice bran, corn powders, starch, glucose, yeast, fish meal, talc, or diatomaceous earth.

7. The efficiency-improving agent for a livestock or poultry feed according to Claim 5 in which the liquid carrier is a physiological saline solution, distilled water, or a phsiologically acceptable organic. solvent.

8. The efficiency-improving agent for a livestock or a poultry feed or a preventive and treating agent for livestock or poultry coccidiosis according to Claim 1, 2 or 3 which contains an emulsifying agent, dispersant, suspending agent, moistening agent, concentrating agent, gelation agent, antimicrobial agent, antiseptic agent, enzyme preparation, antibiotic, or lactic acid preparation, as a supplementary agent or additive.

9. Use of antibiotic 5057A or its physiologically acceptable salt and/or antibiotic 5057B or its physiologically acceptable salt for preparing the efficiency-improving agent for a livestock or poultry feed or the preventive and treating agent for livestock or poultry coccidiosis according to claims 1 to 8.

**Claims for the Contracting State: AT**

1. A process for preparing an efficiency-improving agent for a livestock or poultry feed or a preventive and treating agent for livestock or poultry coccidiosis, characterized in that antibiotic 5057A or its physiologically acceptable salt and/or antibiotic 5057B or its physiologically acceptable salt as an effective ingredient is combined with a physiologically acceptable solid or liquid diluent or is directly incorporated into feed or drinking water.

2. The process according to Claim 1 in which the amount of the effective ingredient is 5—200 ppm.

3. The process according to Claim 1 in which the amount of the effective ingredient is 10—250 ppm.

4. The process according to Claim 1, 2 or 3 in which the feed or agent is produced in the form of powders, tablets, capsules, granules, or pills.

5. The process according to Claim 1, 2 or 3 in which the agent is produced in the form of a premix comprising a purified product or a crude product of the effective ingredient, or cells containing it and a physiologically acceptable solid or liquid carrier.

6. The process according to Claim 5 in which the solid carrier is flour, soybean powders, rice bran, corn powders, starch, glucose, yeast, fish meal, talc, or diatomaceous earth.

7. The process according to Claim 5 in which the liquid carrier is a physiological saline solution, distilled water, or a physiologically acceptable organic solvent.

8. The process according to Claim 1, 2 or 34 in which an emulsifying agent, dispersant, suspending agent, moistening agent, concentrating agent, gelation agent, antimicrobial agent, antiseptic agent, enzyme preparation, antibiotic, or lactic acid preparation is employed as a supplementary agent or additive.

9. Use of antibiotic 5057A or its physiologically acceptable salt and/or antibiotic 5057B or its physiologically acceptable salt for preparing the efficiency-improving agent for a livestock or poultry feed or the preventive and treating agent for livestock or poultry coccidiosis according to claims 1 to 8.

**Patentansprüche für die Vertraggsstaaten: BE CH DE FR GB IT LI LU NL SE**

1. Mittel zur Verbesserung der Wirksamkeit von Vieh- oder Geflügelfutter oder Mittel zur Verhinderung und Behandlung von Vieh- oder Geflügel-Coccidose, gekennzeichnet dadurch, daß es Antibiotikum 5057A oder ein physiologisch verträgliches Salz davon und/oder Antibiotikum 5057B oder ein physiologisch verträglishes Salz davon als wirksamen Bestandteil enthält.

2. Mittel zur Verbesserung der Wirksamkeit von Vieh- oder Geflügelfutter nach Anspruch 1, wobei die Menge des wirksamen Bestandteils 5 bis 200 T.p.M. beträgt.

3. Mittel zur Verhinderung und Behandlung von Vieh- oder Geflügel-Coccidiose nach Anspruch 1, wobei die Menge des wirksamen Bestandteils 10 bis 250 T.p.M. beträgt.

4. Mittel zur Verbesserung der Wirksamkeit von Vieh- oder Geflügelfutter oder Mittel zur Verhinderung und Behandlung von Vieh- oder Geflügel-Coccidiose nach Anspruch 1, 2 oder 3, wobei das Futter oder das Muttel in Pulver-, Tabletten, Kapsel-, Granulat- oder Pillenform vorliegen.

5. Mittel zur Verbesserung der Wirksamkeit von Vieh- oder Geflügelfutter oder Mittel zur Verhinderung und Behandlung von Vieh- oder Geflügel-Coccidiose nach Anspruch 1, 2 oder 3, wobei das Mittel ein Vorgemisch ist, das ein gereinigtes Produkt oder ein Rohprodukt des wirksamen Bestandteils oder dieses enthaltende Zellen und einen physiologisch verträglichen festen oder flüssigen Träger umfaßt.

6. Mittel zur Verbesserung der Wirksamkeit von Vieh- oder Geflügelfutter nach Anspruch 5, wobei der feste Träger Mehl, Sojabohnenpulver, Reiskleie, Maispulver, Stärke, Glucose, Hefe, Fischmehl, Talk oder Diatomeenerde ist.

7. Mittel zur Verbesserung der Wirksamkeit von Vieh- oder Geflügelfutter nach Anspruch 5, wobei der flüssige Träger eine physiologische Kochsalzlösung, destilliertes Wasser oder ein physiologisch verträgliches organisches Lösungsmittel ist.

8. Mittel zur Verbesserung der Wirksamkeit von Vieh- oder Geflügelfutter oder Mittel zur Verhinderung und Behandlung von Vieh- oder Geflügel-Coccidiose nach Anspruch 1, 2 oder 3, das ein Emulgiermittel, Dispersant, Suspendiermittel, Benetzungsmittel, Konzentrationsmittel, Gelierungsmittel, antimikrobielles Mittel, anti-septisches Mittel, eine Enzympräparation, ein Antibiotikum oder eine Milchsäurepräparation als Zusatzmittel oder Additiv enthält.

9. Verwendung von Antibiotikum 5057A oder eines physiologisch verträglichen Salzes davon und/oder Antibiotikum 5057B oder eines physiologisch verträglichen Salzes davon zur Herstellung des die Wirksamkeit von Vieh- oder Geflügelfutter verbessernden Mittels oder des Mittels zur Verhinderung und Behandlung Vieh- oder Geflügel-Coccidiose nach den Ansprüchen 1 bis 8.

**Patentansprüche für den Vertragsstaat: AT**

1. Verfahren zur Herstellung eines Mittels zur Verbesserung der Wirksamkeit von Vieh- oder Geflügelfutter oder eines Mittels zur Verhinderung und Behandlung von Vieh- oder Geflügel-Coccidiose, gekennzeichnet dadurch, daß Antibiotikum 5057A oder ein physiologisch verträgliches Salz davon und/oder Antibiotikum 5057B oder ein physiologisch verträgliches Salz davon als ein wirksamer Bestandteil mit einem physiologisch verträglichen festen oder flüssigen Verdünnungsmittel kombiniert oder direkt dem Futter oder Trinkwasser einverleibt wird.

2. Verfahren nach Anspruch 1, wobei die Menge des wirksamen Bestandteils 5 bis 200 T.p.M. beträgt.

3. Verfahren nach Anspruch 1, wobei die Menge des wirksamen Bestandteils 10 bis 250 T.p.M. beträgt.

4. Verfahren nach Anspruch 1, 2 oder 3, wobei das Futter oder Mittel in Pulver-, Tabletten-, Kapsel-, Granulat- oder Pillenform hergestellt wird.

5. Verfahren nach Anspruch 1, 2 oder 3, wobei das Mittel in Form eines Vorgemisches hergestellt wird, das ein gereinigtes Produkt oder ein Rohprodukt des wirksamen Bestandteils oder dieses enthaltende Zellen und einen physiologisch verträglichen festen oder flüssigen Träger umfaßt.

6. Verfahren nach Anspruch 5, wobei der feste Träger Mehl, Sojabohnenpulver, Reiskleie, Maispulver, Stärke, Glukose, Hefe, Fischmehl, Talk oder Diatomeenerde ist.

7. Verfahren nach Anspruch 5, wobei der flüssige Träger eine physiologische Kochsalzlösung, destilliertes Wasser oder ein physiologisch verträgliches organisches Lösungsmittel ist.

8. Verfahren nach Anspruch 1, 2 oder 3, wobei ein Emulgiermittel, Dispersant, Suspendiermittel, Benetzungsmittel, Konzentrationsmittel, Gelatiermittel, antimikrobielles Mittel, antiseptisches Mittel, eine Enzympräparation, ein Antibiotikum oder eine Milchsäurepräparation als Zusatzmittel oder Additiv verwendet wird.

9. Verwendung von Antibiotikum 5057A oder eines physiologisch verträglichen Salzes davon und/oder Antibiotikum 5057B oder eines physiologisch verträglichen Salzes davon zur Herstellung des Mittels zur Verbesserung der Wirksamkeit von Vieh- oder Geflügelfutter oder des Mittels zur Verhinderung und Behandlung von Vieh- oder Geflügel-Coccidiose nach den Ansprüchen 1 bis 8.

**Revendications pour les Etats contractants: BE CH DE FR GB IT LI LU NL SE**

1. Agent pour l'amélioration de l'efficacité des aliments pour le bétail ou la volaille, ou agent pour la prévention et le traitement de la coccidiose du bétail ou de la volaille, caractérisé en ce qu'il contient l'antibiotique 5057A ou un sel physiologiquement acceptable de ce dernier et/ou l'antibiotique 5057B ou un sel physiologiquement acceptable de ce dernier, à titre d'ingrédient efficace.

2. Agent pour l'amélioration de l'efficacité des aliments pour le bétail ou la volaille suivant la revendication 1, caractérisé en ce que la quantité de l'ingrédient efficace est de 5 à 200 ppm.

3. Agent pour la prévention et le traitement de la coccidiose du bétail ou de la volaille suivant la revendication 1, caractérisé en ce que la quantité de l'ingrédient efficace est de 10 à 250 ppm.

4. Agent pour l'amélioration de l'efficacité des aliments pour le bétail ou la volaille, ou agent pour la prévention et le traitement de la coccidiose du bétail ou de la volaille suivant l'une quelconque des revendications 1, 2 et 3, caractérisé en ce que l'aliment ou l'agent se présente sous la forme de poudres, de comprimés, de capsules, de granules, ou de pilules.

5. Agent pour l'amélioration de l'efficacité des aliments pour le bétail ou la volaille, ou agent pour la prévention et le traitement de la coccidiose du bétail ou de la volaille suivant l'une quelconque des revendications 1, 2 et 3, caractérisé en ce que l'agent est un prémélange comprenant un produit purifié ou un produit brut de l'ingrédient efficace, ou des cellules en contenant, et un support solide ou liquide physiologiquement acceptable.

6. Agent pour l'amélioration de l'efficacité de aliments pour le bétail ou la volaille suivant la revendication 5, caractérisé en ce que le support solide est de la farine, des poudres de soja, du son de riz, des poudres de maïs, de l'amidon, du glucose, de la levure, de la farine de poisson, du talc, ou une terre à diatomées.

7. Agent pour l'amélioration de l'efficacité des aliments pour le bétail ou la volaille suivant la revendication 5, caractérisé en ce que le support liquide est une solution saline physiologique, de l'eau distillée, ou un solvant organique physiologiquement acceptable.

8. Agent pour l'amélioration de l'efficacité des aliments pour le bétail ou la volaille ou agent de prévention ou de traitement de la coccidiose du bétail ou de la volaille suivant l'une quelconque des revendications 1, 2 ou 3, caractérisé en ce qu'il contient un agent émulsionnant, un agent dispersant, un agent de suspension, un agent mouillant, un agent de concentration, un agent gélifiant, un agent antimicrobien, un agent antiseptique, un préparation enzymatique, un antibiotique, ou une préparation d'acide lactique, comme agent ou additif supplémentaire.

9. Utilisation de l'antibiotique 5057A ou d'un sel physiologiquement acceptable de ce dernier et/ou de l'antibiotique 5057B ou d'un sel physiologiquement acceptable de ce dernier, dans la préparation de l'agent d'amélioration de l'efficacité des aliments pour le bétail ou la volaille, ou de l'agent de prévention ou de traitement de la coccidiose du bétail ou de la volaille, suivant l'une quelconque des revendications 1 à 8.

**Revendications pour l'Etat contractant: AT**

1. Procédé de préparation d'un agent pour l'amélioration de l'efficacité des aliments pour le bétail ou la volaille, ou d'un agent pour la prévention et le traitement de la coccidiose du bétail ou de la volaille, caractérisé en ce que l'antibiotique 5057A ou un sel physiologiquement acceptable de ce dernier et/ou l'antibiotique 5057B ou un sel physiologiquement acceptable de ce dernier sont, à titre d'ingrédient efficace, combinés avec un diluant solide ou liquide, physiologiquement acceptable, ou directement incorporés aux aliments ou à l'eau potable.

2. Procédé suivant la revendication 1, caractérisé en ce que la quantité d'ingrédient efficace est de 5 à 200 ppm.

3. Procédé suivant la revendication 1, caractérisé en ce que la quantité d'ingrédient efficace est de 10 à 250 ppm.

4. Procédé suivant l'une quelconque des revendications 1, 2 et 3, caractérisé en ce que l'aliment ou l'agent est produit sous la forme de poudres, de comprimés, de capsules, de granules, ou de pilules.

5. Procédé suivant l'une quelconque des revendications 1, 2 ou 3, caractérisé en ce que l'agent est produit sous la forme d'un prémélange comprenant un produit purifié ou un produit brut de l'ingrédient efficace, ou des cellules en contenant, et un support solide ou liquide, physiologiquement acceptable.

6. Procédé suivant la revendication 5, caractérisé en ce que le support solide est de la farine, des poudres de soja, du son de riz, des poudres de maïs, de l'amidon, du glucose, de la levure, de la farine de poisson, du talc ou une terre à diatomées.

7. Procédé suivant la revendication 5, caractérisé en ce que le support liquide est un solution saline physiologique, de l'eau distillée ou un solvant organique physiologiquement acceptable.

8. Procédé suivant l'une quelconque des revendications 1, 2 et 3, caractérisé en ce qu'un agent émulsionnant, un agent dispersant, un agent de suspension, un agent mouillant, un agent de concentration, un agent gélifiant, un agent antimicrobien, un agent antiseptique, une préparation enzymatique, un antibiotique ou une préparation d'acide lactique est utilisé comme agent ou additif supplémentaire.

9. Utilisation de l'antibiotique 5057A ou d'un sel physiologiquement acceptable de ce dernier et/ou de l'antibiotique 5057B ou d'un sel physiologiquement acceptable de ce dernier pour la préparation de l'agent d'amélioration de l'efficacité des aliments pour le bétaile ou la volaille, ou de l'agent de prévention et de traitement de la coccidiose du bétail ou de la volaille, suivant l'une quelconque des revendications 1 à 8.